# EUROPEAN PATENT APPLICATION

(11) **EP 0 997 123 A1**
(43) Date of publication of application: **03.05.2000**
(21) Application number: 98830659.3
(22) Date of filing: 30.10.1998
(51) Int. Cl.: A61F 13/15

(54) **Method and device for applying laminar elements onto a substrate**

(71) Applicant: Fameccanica. Data S.p.A., 65127 Pescara (IT)
(72) Inventor: Pasqualoni, Paolo, 66020 Sambuceto di san Giovanni Teatino (IT)
(74) Representative: Bosotti, Luciano

(57) **Abstract**

Laminar elements (5) are applied to a substrate (1) moving in a given direction (X1), the said laminar elements extending in an inclined direction with respect to the said given direction. The laminar elements are cut from a strip (R) extending in the said given direction, in a cutting operation performed in an orthogonal direction to the said given direction. The laminar elements (5) formed by the said cutting operation are received by a transfer support (10) which, at least at a transfer position, moves in concordance with the said substrate. The laminar elements (5) received by the said transfer support (10) are orientated from the cutting position orthogonal to the said given direction, to an inclined orientation position (α) with respect to the said given direction, for application to the said substrate (1).

## Description

The present invention generally concerns the problem of applying laminar elements to a substrate, and has been developed with particular attention to its possible application to the manufacture of absorbent articles, preferably of the single-use type (for example, nappies and underwear for babies and children, and products similar thereto such as so-called incontinence pants etc.).

In the currently most widely used embodiment, these articles essentially comprise an elongate body with a layered structure (the basic configuration usually provides for a layer of absorbent material between a liquid-impermeable lower sheet or backsheet and an upper sheet or topsheet that is completely or locally permeable to liquids, plus further layers and accessory elements) intended to assume a general U-shape or shell shape about the crutch region of the wearer's body. Once in place, the article is held in the closed position by so-called tabs having a base or proximal end attached to the rear part of the article, and a front or distal end intended to be attached (by adhesion or other coupling arrangements, for example, a hook and loop structure - of the type currently known as "Velcro") to the front part of the article. To this end, the article itself can have constituent attachment elements for the tabs on its outer front surface (therefore, one or more reinforced areas on the backsheet - known in the prior art by the terms "landing strip" or "frontal tape").

The technical and patent literature concerning articles of the type indicated above, the technology of the associated materials and the corresponding manufacturing techniques is important, especially when one considers the extensive use made of the articles in question.

In the most current arrangements, the tabs are formed as rectangular elements the longest sides of which are disposed exactly orthogonal with respect to the principal direction of extent of the absorbent article (reference will be made in the following description to an article in the form of a nappy for children, without this being interpreted as a limitation on the scope of the invention). Even when it is shaped (for example, to give the article an overall hour-glass shape), the articles still have a longitudinal principal direction of extent.

This direction also corresponds to the direction in which - in the most currently used arrangement - the articles are manufactured in a continuous manufacturing process. Substantially (as has been said, the related technical and patent literature is very extensive), the various layers of material constituting the sanitary article are formed from strips or webs that are unwound from related supply rolls towards one or more work stations. Here, the aforesaid strips, which advance in the aforesaid longitudinal direction, are joined together to form the layered structure of the nappy. The accessory elements, such as the tabs, the frontal strip and possible longitudinal and/or transverse elastication strips, are applied - always operating continuously - to the composite strip or web obtained in this way, or during formation. This leads to the formation of a continuous chain of sanitary articles that are then separated - to form the individual articles - by means of a cutting operation carried out orthogonal to the aforesaid principal direction of extent of the articles themselves.

The orientation of the tabs and/or the corresponding attachment elements (landing tape, frontal strip etc.) in a direction orthogonal to the aforesaid principal direction is certainly advantageous from a production point of view, and is satisfactory for the user.

However, more recent studies demonstrate that this orientation of the tabs (and of the associated attachment parts, if present: the evidence shows that it is preferable for the orientation of the tabs and the attachment parts to be complementary) does not correspond with the arrangement - which is ergonomically ideal, in that it is linked to the U-shape or shell-shape assumed by the article about the wearer's body - that can be achieved if the tabs/attachment parts extend in an inclined direction with respect to the longitudinal axis of the article, in particular, so that the distal part of the tabs is gradually spaced from the corresponding end edge of the article.

In this sense, the document US-A-4 209 016 describes the use of Y-shape adhesive tabs having a main part intended to be connected to the rear part of a nappy, and at least one head part having an inclined branch.

The document US-A-5 358 500 describes the use of tabs having a "straight" proximal or main part (that is, substantially orthogonal to the longitudinal direction of extension of the nappy) and a free or distal part, which is inclined and has a rhombus or rhomboidal shape that assists in its application to a generally V-shape frontal attachment element.

The document US-A-5 603 794, on which the preambles of the following independent claims are based, describes a substantially similar arrangement in which closure tabs having a generally rhomboid shape are used. Here, the inclination of the tab with respect to the principal direction of the nappy is obtained by inclining the cut of the tab. The associated method for application to the nappy continues, however, in the conventional way, that is, in a continuous process. However, this arrangement has problems that are not easy to solve, since the oblique cut presupposes, at least for some tab applying machines, the use of a particular material having isotropic mechanical characteristics, which is difficult to get in the strips from which the currently used tabs are manufactured. The document US-A-5 197 958 describes a re-usable nappy with two attachment elements (in the form of strips of a hook and loop tape) disposed in a generally inclined direction with respect to the principal axes of the nappy. The patent does not provide further details concerning the method of application, limiting itself to making reference to hook and loop element sewn to the nappy.

The object of the present invention is therefore to provide an arrangement that overcomes the disadvantages of the prior art arrangements thus enabling the application, using the continuous manufacturing methods that have been widely tested in the past, of laminar elements such as tabs and/attachment elements to sanitary articles. For this purpose, currently available materials are used (for example, laminated plastics materials, hook and loop closures etc.), avoiding the need for materials having certain characteristics (mechanical isotropy, etc.).

According to the present invention, this object is achieved by virtue of a method having the further characteristics referred to in Claim 1. The invention also concerns a device for performing the aforesaid method as well as an associated sanitary article.

The invention will now be described, purely by way of non-limitative example, with reference to the accompanying drawings, in which:
- Figure 1 is a schematic view of a sanitary article produced according to the invention;
- Figure 2 illustrates schematically the method for applying the tabs/attachment elements to a sanitary article;
- Figure 3 is a general perspective view of part of the device according to the invention with various parts removed for clarity of illustration;
- Figure 4 is a diametrally opposite view of the invention illustrated in Figure 3;
- Figure 5 is a section taken on the line V-V of Figure 4; and
- Figure 6 is a view in section taken on the line VI-VI of Figure 5.

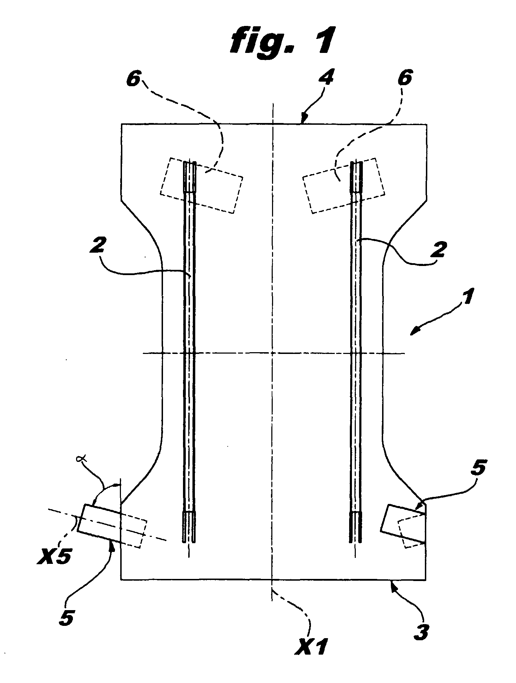

In Figure 1, the reference numeral 1 indicates an absorbent article such as a nappy for a child. According to one widely known arrangement, the article 1 includes a so-called upper sheet or topsheet that is permeable to liquids, a so-called base sheet or backsheet that is impermeable to liquids and connected to the topsheet, and an absorbent core located between the topsheet and the backsheet.

The nappy 1, which has a general hour-glass shape (but, as is well known to the expert in the field, this shape could be entirely different) preferably has longitudinal elastication elements 2 intended to obtain the optimum adherence of the nappy 1, when worn, to the contours of the wearer's body. Transverse elastication elements may be present, even though they are not explicitly illustrated (especially at the front and rear ends, in order to assist in a greater adherence to the shape of the wearer's body), as well as further elastication elements intended to lift the sides of the topsheet in order to provide a greater containment action of the exudatiory and/or the excretions that the nappy is intended to receive.

It will also be appreciated that the arrangement according to the invention finds application irrespective of the specific manufacturing characteristics of the article to which it is applied.

In Figure 1, the nappy 1 has been represented in its unfolded state as theoretically seen from the inside, that is, the side intended to face the wearer. In other words, the side of the nappy where the topsheet is located.

In relation to the shape of the nappy, two ends 3 and 4 are distinguishable, respectively, front and rear in the normal condition in which the nappy is worn.

Two attachment elements 5, usually called "tabs", are present at the rear end of the nappy, intended to enable the closure of the article 1 about the wearer's body.

Usually, in the nappy 1 as provided to the user, the tabs 5 are closed in a V-shape with the two branches, or proximal and distal portions, located in correspondence with the backsheet and topsheet respectively. This is the condition represented in Figure 1 with reference to the tab 5 on the right hand side. However, as is also known to the man skilled in the art, the tab can, in reality, have a complex structure in order to take account of the requirements of ensuring the secure anchorage to the rear part of the nappy, and ensuring the easy detachment, during use, of the distal portion which is initially in a folded position.

In use, the tabs 5 are unfolded by the user to bring them to the condition shown with reference to the tab 5 on the left in Figure 1. This is the same unfolded condition in which the tabs 5 are usually attached to the body of the nappy 1 during the manufacturing process, as will be better illustrated below. However, as is known to the man skilled in the art, manufacturing arrangements exist in which the tabs are attached to the nappy in an at least partly closed condition. In any case, it will be appreciated that the arrangement according to the invention also lends itself to application to these different modes of application.

According to an important characteristic of the arrangement according to the invention, the tabs 5 (which, in the embodiment illustrated here, are generally rectangular in shape) are disposed with their longest sides not quite orthogonal with respect to the principal longitudinal axis X1 of the nappy 1, but orientated in a generally inclined direction according to an angle α of the order of, for example, 70-80°.

The direction of inclination is such that the proximal portion of the tabs 5 (that is, the portion attached to the body of the nappy 1) is closer to the corresponding end (the end 3) than the distal portion of the same tab, intended to be connected to the front end of the nappy 1 when worn.

The term "connected", as used in the present description and, where appropriate, in the following claims, is naturally intended in its widest sense. Even though reference is made in the following description to an arrangement - currently preferred - in which the tabs 5 are essentially adhesive tabs intended to be connected adhesively to the front end of the nappy 1, the arrangement according to the invention can be effected using completely different connection means. For example, the tabs 5 can be formed, entirely or in part, from hook and loop elements (preferably of the male type, but they can also be female-type elements), for example, of the type currently known as "Velcro". In addition, a completely different arrangement from that illustrated can, of course, be hypothesised for the tabs 5, for example, where the tabs are located on the front part of the nappy 1 and are intended to be closed onto the rear part of the nappy.

As already indicated above, and as will be seen better below, an important characteristic of the invention is the fact that a normal material that is currently utilised in the prior art for this purpose can be used to manufacture the tabs 5, in particular, a non-isotropic material in the sense that it has, for example, a direction (identified in Figure 1 by the axis X5) of maximum or greater resistance to stresses.

As is known, the presence of a preferable direction of stress resistance can arise, in the case of plastics and laminated films (possibly adhesive and/or that can be made adhesive), from the manufacturing method thereof. In the case of tabs 5 manufactured using different techniques (for example, techniques similar to those used in the textile sector, such as certain hook and loop tabs), the axis X5 can correspond, for example, to a direction of one of the threads in the textile or para-textile substrate of the associated material.

In any case, the axis X5 is located, with respect to the principal axis X1 of the nappy 1, according to the angle α indicated above. At the same time, the arrangement according to the invention enables, for the manufacture of the tabs, the widely tested technique of forming the tabs by cutting a strip transversely of the principal direction of the strip itself, which also corresponds to the direction of maximum stress resistance, to be used. The difference is clear on comparing the arrangements of patents US-A-5 358 500 and US-A-5 603 794, in which the tabs are formed, entirely or in part, by cutting in the oblique direction.

Similar or substantially similar considerations to those relating to the tabs 5 apply to the attachment elements 6 provided on the opposite end of the nappy 1, usually (but not necessarily) on the outer surface of the backsheet.

In the embodiment illustrated, the attachment elements 6 in question are formed simply from two strips forming two so-called "landing strips" to which, once the nappy 1 has been placed on the wearer and shaped thereto, the tabs 5 can be connected (for example, adhesively) to keep the nappy closed.

In the particular embodiment illustrated, the two attachment elements 6 are rectangular and are also arranged with their longest sides inclined with respect to the principal axis X1 of the nappy 1.

The angle can be identical to or different from that of the tabs 5, depending on the specific requirements of use.

As is well known to the man skilled in the art, the presence of the attachment elements 6, in particular in the form illustrated here, is not essential. The attachment elements 6 usually have the function of strengthening, at least locally, the front part of the nappy 1 so that it can withstand without tearing the stresses to which it is subjected by the tabs 5 during use, and/or to co-operate with the tabs in achieving a re-positionable connection. This is particularly relevant when it is wished to position and re-position the tabs 5, even in different locations, for example, to open the nappy momentarily or correct an unsatisfactory attachment.

The attachment elements could, however, be constituted from a single strip (a so-called "frontal tape") extending transversely along all or nearly all of the length of the nappy 1. Alternatively, two elements like the elements 6 illustrated in the example of Figure 1 could be longer and thus connect together at the principal axis X1 of the nappy to form a single, generally V-shape attachment element (see, for example, the arrangement described in the document US-A-5 358 500).

In addition, the nappy 1 could be entirely free of attachment elements of the type illustrated if the degree of stress resistance of the material forming the backsheet is sufficient for the intended conditions of use, and this is so in the case of adhesive tabs, as well as where the tabs themselves have a hook and loop surface that can be attached directly to the material of the backsheet or a covering applied thereto.

It is therefore clear that the arrangement according to the invention lends itself, in general, to resolving the problem of applying - in the ambit of a mass production process typical, for example, of hygienic-sanitary articles such as nappies - one or more components of the article that extend, with their direction of principal action (usually coincident with the direction of greatest extent and/or greatest stress resistance) in an oblique position, thus neither parallel nor orthogonal, with respect to the principal direction of the articles in question, usually coincident with the direction in which the mass production occurs.

The description below therefore refers specifically to the problem of applying one of the oblique elements constituted, in the embodiment illustrated, by the two tabs 5 and the attachment elements 6, to a product such as a nappy 1.

The arrangement described with reference to one element can be multiplied to enable the application of any number n of inclined or oblique elements.

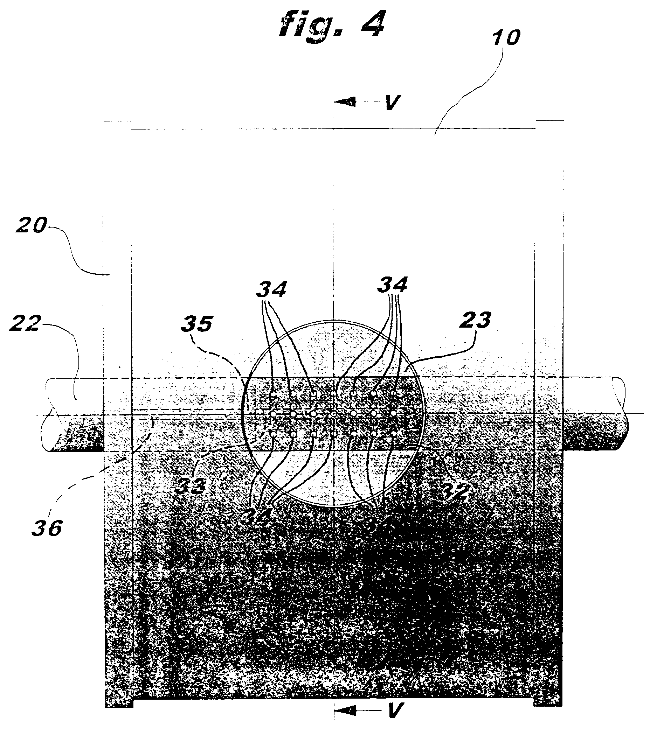

For example, and still with reference to the nappy 1, the arrangement described below with reference to Figures 4 to 6 can be reproduced in a quadruple applicator structure for applying simultaneously or almost simultaneously two pairs of inclined elements, that is, the two tabs 5 and (if present in the embodiment illustrated) the elements 6.

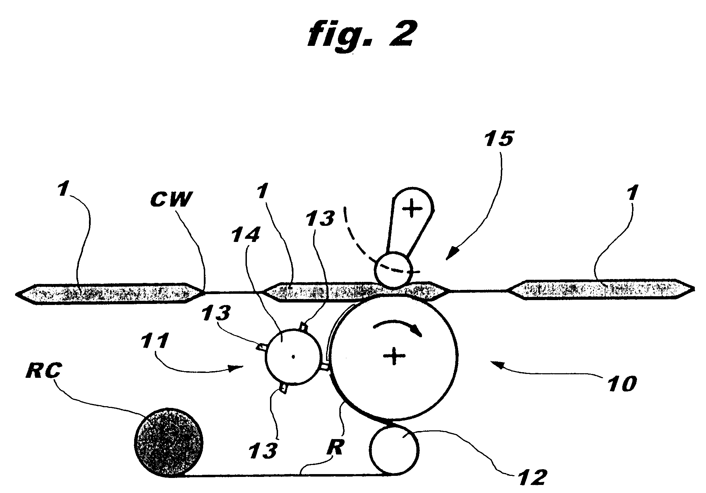

Figure 2 generally illustrates the criteria (in themselves known, and therefore not requiring detailed illustration here) usually adopted for applying accessory elements such as, for example, the closure tabs 5 (or one or more attachment elements such as the elements 6) to a continuous strip or web CW constituted by a chain of sanitary articles such as the nappies 1 in the course of formation that have not yet been separated into single articles.

Substantially, the continuous strip or web CW advances (from left to right, with reference to Figure 2 of the accompanying drawings) close to a drum 10 that turns (in a clockwise direction, still with reference to Figure 2) in order to transfer laminar elements such as the tabs 5 onto the individual articles 1 in the strip CW.

The tabs 5 in question (by way of example, reference will be made below to the tabs 5, but this discussion applies equally to any similar element, such as the attachment elements 6) are usually obtained from a further strip R by means of a cutting operation performed in a cutting station indicated 11.

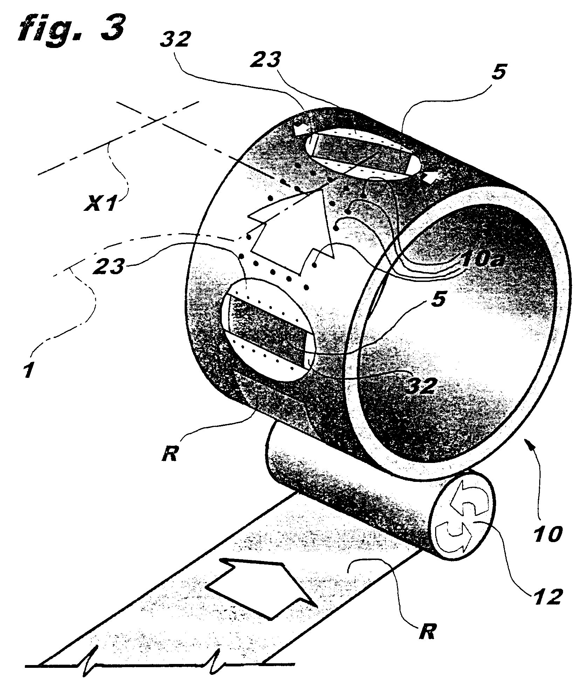

Preferably, the strip R is unwound from an associated supply reel RC, and advances towards the drum 10, moving in contact with the surface of the drum 10 to wind around a guide roller 12 tangential to the drum 10. This is so that the further strip R is brought to the position in which the cutting station 11 operates (not shown in Figure 3 for clarity of representation) in contact with the outer surface or cladding of the drum 10 itself.

The cutting station 11 usually includes a rotary knife having at least one blade 13, preferably mounted on an associated rotary element 14. Preferably, this element has a plurality of cutting blades 13 (four, separated by 90°, in the embodiment illustrated) that act on the strip R adhering to the cladding surface of the drum 10 so as to segment it into pieces of predetermined length: each of these pieces forms one of the tabs 5. It will be appreciated that the aforesaid cutting action is effected in a direction orthogonal to the direction of unwinding of the strip R.

After having been separated from the strip R, the tabs 5 advance, held against the surface of the drum 10, until they reach an application station 15 where they are deposited on to the surface of the articles 1, connecting thereto.

The connection can occur in different ways (gluing by introducing gluing material, ultrasonic welding, mechanical coupling etc.). A current arrangement for producing the application stations 15 is using an application roller (or any similar movable element) which co-operates with the drum 10 in order to form a nip in the space between the roller of the application station 15 and the drum 10, in which the tabs (or, in any case, the applied elements) are pressed against the strip CW, achieving the desired connection.

Among other things, the structure of the strip CW can correspond to the structure of the finished article 1 (this can be so, for example, for the attachment elements 6 applied to the outer surface of the backsheet of an article such as the nappy 1) or the structure of an intermediate product intended to be completed with the addition of further parts following the application of the elements in question.

The various moving elements described above (that is, the continuous strip CW, the strip R, the drum 10, the roller 12, the cutting station 11, the application station 15 etc.) are motorised by means, not illustrated, that are usually controlled (directly and/or by a single control unit such as a so-called PLC or Programmable Logic Controller) to ensure the necessary synchronisation of the velocities and phases of movement. This is so that, at the application station 15, the tabs 5 advancing on the outside of the drum 10 reach the strip CW at a tangential velocity corresponding to the linear velocity of advancement of the strip CW itself in the exact position (phase) as the single article. The velocity of intervention of the cutting station 11 is controlled to produce a single piece of strip R (that is, a tab 5) with a rhythm corresponding to the application requirements, that is, in the embodiment illustrated, in order to make a tab 5 available at the respective end of an article 1. Similarly, the supply velocity of the strip R is controlled to achieve the correct supply of the material necessary for the cutting station 11. All of the above is achieved according to criteria that are known in the art, so that they do not require specific illustration here. This applies in particular to the requirement that the strip R can slide over the surface of the drum 10, which turns at a tangential velocity usually equal to the "line" velocity, that is, the velocity at which the chain of nappies 1 advances.

However, it will be appreciated that both the advancement of the strip CW of articles 1, as well as the application of the tabs 5 to these articles, occurs in line, that is, on parts that advance together in a single direction (corresponding to the direction of the plane of the sheet, with reference to Figure 2). However, a similar result would not be obtainable if one were to attempt to apply the tabs 5 by advancing them towards the strip CW in an oblique direction with respect to the longitudinal axis of the strip CW itself, for example, positioning known tabling machines with their axis of application oblique with respect to the axis of the strip. In this case, it would cause, especially when operating at high velocity, significant relative sliding on application.

An important characteristic of the arrangement according to the invention is that, contrary to the conventional systems, during the movement on the surface of the drum 10 between the angular position at which the cutting station 11 acts and the position of application to the strip CW, the tabs 5 are rotated. This movement aims to achieve, although being obtained by cutting the strip R transversely (in particular, with a cutting action performed in a direction orthogonal to the longitudinal direction of extension and advancement of the strip R), that the tabs 5 are applied to the strip CW in an angled position (the angle α of Figure 1) with respect to the principal direction of advancement of the strip CW of articles 1.

This is illustrated schematically in the upper part of Figure 3 in which the shape of the adjacent head parts of two successive nappies 1 in the chain represented by the strip CW has been represented in broken outline.

Essentially, the drum 10 is formed as a cylindrical structure having a perforated wall (holes 10a are present at least in a central portion of the cladding surface of the drum 10) according to a structure that is more or less the same as that of a washing machine drum.

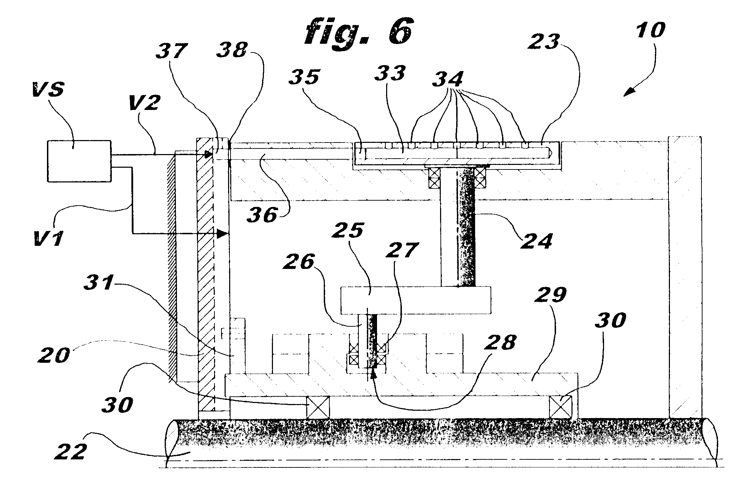

End seal elements 20 are associated with the cladding wall in question (see Figure 6), intended to function such that, although it is rotated about its principal axis by a drive shaft 22 (operated by a motor, not shown), the cladding surface of the drum 10 can be maintained at a level of sub-atmospheric pressure (a vacuum). This is to ensure that the portion of strip R extending from the roller 12 towards the cutting station 11 is held to the cladding surface in question (although being free to slip thereon) and, at the same time, once cut from the body of the strip R, the tabs 5 are held to the surface of the drum 10 (and, in particular, to the portion thereof that, according to the method described below, determines the orientation of the tabs 5) until they are applied to the strip CW at the station 15.

The manufacture of a perforated drum structure (which can have soft walls) with associated aspiration means for the establishment of pressure gradients intended to hold a strip in contact with the drum is well known in the art.

At least one and preferably a plurality of rotatable inserts 23 are present on the cladding surface of the drum 10, the function of which is to remove the tabs 5 at the moment at which they are cut from the strip R in order then to orientate them as described above.

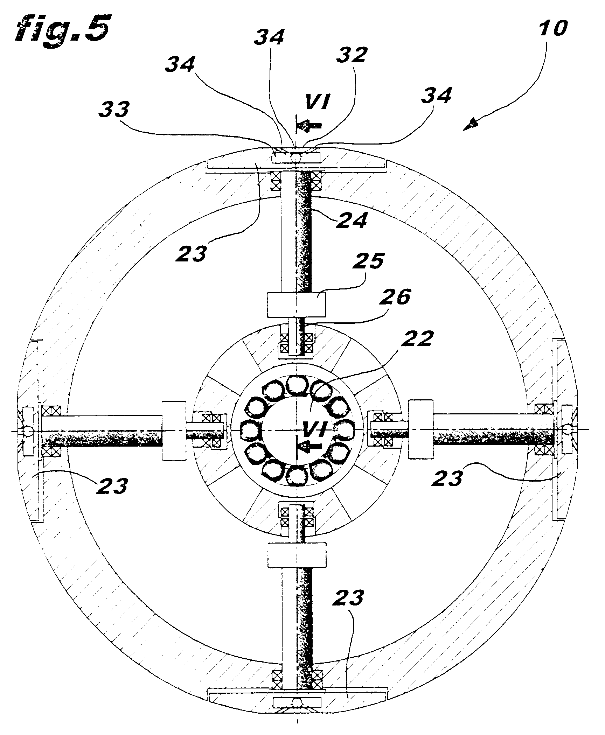

As will be better appreciated from the sectional views of Figures 5 and 6, the or each insert 23 (there are four inserts 23 in the embodiment illustrated in the accompanying drawings, but this choice is not in any way limiting) form the head part of a mushroom-shape structure, the leg 24 of which extends radially into the drum 10, terminating in a crank arm 25.

At its free end, distal with respect to the shaft 24, the crank arm 25 carries a pin 26 that co-operates, preferably with interposed revolving means such as one or more bearings 27, with a hollow 28 formed in a sleeve body 29. The sleeve 29 surrounds, in this case also, with similar bearings 30 preferably interposed, the shaft 22, therefore maintaining a fixed position with respect to the structure of the drum 10. For example, the ends of the sleeve body 29 can be fixed, possibly with the interposition of a flange 31, to one of the end bodies 22 forming the seal of the vacuum chamber present within the drum 10.

During operation, the drum 10 turns and with it turn the inserts 23, the associated shafts 24 and the crank arm 25. The pins 26 carried by these latter therefore orbit within the channel defined by the hollow 28 in the manner of a general cam mechanism. The cam defined by the hollow 28 is shaped (in known way) so that, when each element reaches, due to the rotation of the drum 10, the position corresponding to the zone in which the cutting unit 11 operates, the crank arm 25 is in an angular position so that the shaft 24, and therefore the insert 23 carried thereby, assume a predetermined angular position, such as that illustrated in the first plane in Figure 3. Then, gradually, due to the rotation of the drum 10 (in the clockwise direction with reference to Figure 3), the respective element 23 advances towards the zone where the application unit 15 is located, the arm 25 rotates the shaft 24 and the associated insert 23 with an angular movement the magnitude of which is chosen so as to orientate the tab 5 carried by the insert 23 to a predetermined extent (10-20°, typically around 15°, in practice, greater than 90⁰-α). This is to ensure that the tab 5 mounted on the insert 23 arrives at the strip C orientated obliquely (according to the angle α in Figure 1) with respect to the general direction of advancement identified by the axis X1.

Once the tab 5 has been transferred to the strip CW, the associated insert 23 can then be returned to the angular starting position in order to re-present itself at the position at which the tabs 5 are removed, where the cutting unit 11 acts, in the predetermined angular position described above.

By modifying the profile of the hollow 28, acting as a cam, it is possible to vary selectively the magnitude of the rotation of the inserts 23, and therefore vary selectively the angle of inclination at which the tabs 5 are applied to the strip CW, and therefore to the articles 1.

It will therefore be appreciated that, although the tabs 5 are orientated according to the desired angle α, they are applied to the strip CW in conditions without any relative movement. At the moment of arrival at the application station 15, the (tangential) velocity vector of the tabs 5 is, in fact, exactly parallel, in the same in direction and identical with respect to the velocity vector of the strip CW.

This result is achieved without in any way altering the characteristics of the tabs C, in particular as regards the possible anisotropy of the mechanical characteristics thereof.

A comparison of Figure 5 and 6 leads one to understand that the inserts 25, while being generally disc shape, in reality have an approximate tile shape, with a gradual curvature so that, in the angular position in which the inserts 23 arrive at the cutting station 11 (a position referred to in Figures 4 and 5), the axis of the general cylindrical jacket of the outer surface of the inserts 23 is substantially coincident, or otherwise parallel, to the principal axis of the drum 10 and therefore the axis of the shaft 22.

Considering Figure 5 in particular, it can thus be noted that at the central part, that is, in alignment with the shaft 24 and the radial direction of the drum 10, each insert 23 has a substantially flat front surface 32 for receiving the tab.

The presence of this flat surface is intended to facilitate the cutting operation by the blades 13 of the unit 11: in practice, the or each insert 23 (and especially its flat front surface 32) is intended to act as an anvil in the presence of the blades 13 of the unit 11 that cut the strip R. At the same time, the fact that the element formed as the result of the cutting operation is applied to and held on the flat surface 32 assists in the successive transfer and application to the receiving substrate (the strip CW).

Preferably, a cavity 33 is present in the body of each insert 23, from which a plurality of ducts run to the outer surface of the insert 23, especially the flat surface 32, where there are associated apertures 34.

In the embodiment illustrated here, there are three parallel lines of holes or apertures that extend practically over most of the flat front surface 32 of the insert 23.

At the opposite end, the cavity 33 has an enlarged part 35 that extends just below the outer surface of the drum 10 in a position facing a duct 36 connected (according to known criteria) to the source that produces the vacuum within the drum 10.

Preferably, the source in question (typically constituted by a vacuum pump, of known type, and indicted schematically in the drawings with the block VS in Figure 6) communicates with the drum 10 through two distinct lines indicted V1 and V2, respectively.

The line V1 communicates essentially with the interior of the drum 10 and is intended to maintain a vacuum therein such that, through the apertures 10a on the surface of the drum 10 - outside the zone of the inserts 23 - a gas pressure gradient directed inside the drum 10 is established, and so that the pieces of the strip R between the roller 12 and the cutting unit 11, although being held in contact with the cladding surface of the drum 10, are free to slide thereon. It is, in fact, clear that the supply velocity, usually stepwise, of the strip R to the cutting unit 11 is a lot less than the tangential velocity of the outer surface of the drum 10.

On the other hand, the second vacuum line V2 leads to the line 36 associated with the or each insert 23 in order to establish within the cavity 33, and therefore in correspondence with the apertures 34, a vacuum level that is more marked (or, in absolute terms, a lower absolute pressure) than that within the drum 10. In this way, a firm retention action is formed at the inserts 23, in the presence of the tabs 5, that is directed to avoiding a situation in which the tabs 5 can accidentally move with respect to the inserts 23 during their transfer from the cutting unit 11 to the application unit 15. Such a movement could lead to their application to the strip CW in a position different from that envisaged, in particular, with a different angle of inclination from the required angle α.

It will therefore be appreciated that, even though the arrangement described here presupposes a sub-atmospheric pressure level within the entire drum 10, it is in fact sufficient to create this condition only in the angular portion between the region in which the strip R is carried from the roller 12 in contact with the drum 10, and the angular position in which the application unit 15 operates.

Substantially analogous considerations also apply to the vacuum lines V2 and the assembly of parts indicated 33 to 36 that lead to the apertures 34. In this case, it can be useful if the strong vacuum retention action starts to be exerted on the tabs 5 at the zone in which the cutting unit 11 operates and continues until the angular position in which the application unit 15 operates, becoming less strong in correspondence with this unit. This arrangement has the advantage of assisting the transfer of the tabs 5 to the strip CW.

The establishment of sub-atmospheric pressure conditions within the drum for just an angular portion of the peripheral extent thereof can be effected - according to widely known criteria, that do not therefore require detailed illustration here - with the provision of aspiration structures within the drum 10. However, as regards the adoption of a similar configuration in relation to the inserts 23, the same result can be obtained simply by ensuring that the line V2 leads to a manifold 37 formed on the surface of the sealing element 20, facing into the drum 10 and extending along an arcuate path over an arc the size of which corresponds to the size of the portion that the outer end of the duct 36 faces along the path that the or each insert 23 follows from the cutting station 11 to the application station 15.

The seal between the manifold 37 and the end of the duct 36 can be ensured by a sealing element such as a gasket indicated 38 (Figure 6).

The angular extent of the manifold 37 practically corresponds to approximately 90° which, with reference to the presence (although not essential) of four inserts 23 is such that, for each insert 23 at the application station 15, the connection to the vacuum line V2 is cut off, so that the duct 36 and the aspiration elements leading thereto return to substantially atmospheric pressure, with the consequent release of the tab 5 from the drum 10. This occurs while the insert 23 immediately downstream (with respect to the rotation of the drum 10) is facing the duct 36 at the end of the manifold 37 so that the cavity 33 and the apertures 34 leading thereto are affected by the pressure gradient, thus ensuring that the tab 5 that has just been formed by the cutting unit 11 cutting the strip R is firmly retained.

Naturally, the principle of the invention remaining the same, the details of manufacture and the embodiments can be widely varied with respect to that described and illustrated, without by this departing from the ambit of the present invention, as defined in the accompanying claims. This applies, for example, to the structure and the control of the movement of the inserts 23 that orientate the tabs. For example, the movement of these inserts can be controlled using different arrangements from the cam mechanism described above. One of many possible alternative arrangements is the use of a conical wheel mechanism located within the drum 10, which ensures the desired angular movement of the inserts between the position in which the tabs 5 are removed and the position in which they are applied to the strip CW. In particular, it is possible to envisage using a mechanism that does not require the return of the inserts 23 to a predetermined angular position that depends on the removal of the tabs. It is, in fact, sufficient that the inserts 23, once the tab has been removed to a certain relative angular position, ensure the required rotation to the position of application to the strip CW. In this case, it can be appropriate to consider the use of inserts 23 having a generally flat upper surface.

## Claims

1. A method for applying to a substrate (CW) moving in a given direction (X1) laminar elements (5), which extend in a direction (X5) inclined (α) with respect to the said given direction, the said laminar elements being cut (11) from a strip (R) extending in the said given direction, characterised in that it includes the stages of:
- cutting (11) the said laminar elements (5) from the said strip (R) in a cutting operation (13) performed orthogonal to the said given direction (X1),
- receiving the laminar elements (5) formed by the said cutting operation on a transfer support (10) which, at least at a transfer position (15) of the laminar elements (5) to the said substrate (CW), is movable in a movement that is in concordance with the movement of the said substrate (CW),
- transferring the said laminar elements (5) received on the said transfer support (10) to the said transfer portion, orientating them from the said cutting position orthogonal to the said given direction to an inclined orientation (α) with respect to the said given direction, and
- applying (15) the said laminar elements (5) to the said substrate (1) in the said inclined orientation with respect to the said given direction.

2. A method according to Claim 1, characterised in that it includes the operation of choosing the material of the said strip (R) with a direction (X5) of stress resistance orthogonal with respect to the said given direction, so that the said laminar elements (5) are applied to the said substrate (CW) with the said direction of stress resistance (X5) inclined with respect to the said given direction.

3. A method according to Claim 1 or Claim 2, characterised in that the said strip (R) is formed from laminated plastics material.

4. A method according to Claim 1 or Claim 2, characterised in that the said strip (R) is formed from hook and loop material.

5. A device for applying to a substrate (CW) moving in a given direction (X1) laminar elements (5), which extend in a direction (X5) inclined (α) with respect to the said given direction, the device including cutting means (11) for cutting the said laminar elements (5) from a strip (R) extending in the said given direction, characterised in that:
- the said cutting means (11) act (13) on the said strip (R) in a direction orthogonal to the said given direction,
- a transfer support (10) is provided to receive the said laminar elements (5) cut by the said cutting means (11), and transfer them to the said substrate (CW) at a transfer position in which the said transfer support (10) is movable in a movement that concords with the movement of the said substrate (CW),
- orientation means (23) are provided on the said transfer support (10) for orientating the said laminar elements (5), taking them from the said cutting position in a direction orthogonal to the said given direction to an inclined position with respect to the said given direction at the said transfer position, and
- a transfer unit (15) is provided for transferring the said laminar elements (5) orientated in an inclined condition with respect to the said given direction from the said transfer support (10) on the said substrate (CW).

6. A device according to Claim 5, characterised in that the said cutting means (11) include a rotary knife (13, 14).

7. A device according to Claim 5 or Claim 6, characterised in that the said transfer support includes a drum (10) that rotates about a respective axis (22) and receives the said strip (R) at a tangent, and exposes the strip (R) itself to the said cutting means (11) in order then to transfer the laminar elements (5) cut from the said strip (R) to the said substrate substantially at a tangent between the substrate (CW) itself and the surface of the said drum (10).

8. A device according to any of Claims 5 to 7, characterised in that the said transfer support (10) has associated means for generating sub-atmospheric pressure (VS, V1, V2, 33-37) in order to generate, across a surface of the said transfer support (10), a pressure gradient in order to hold the said strip (R) and the said laminar elements (5) in contact with the transfer support (10) itself.

9. A device according to Claim 7 and Claim 8, characterised in that the said sub-atmospheric pressure generating means include at least one sub-atmospheric pressure line (V1) for applying a level of sub-atmospheric pressure within the said drum (10).

10. A device according to any of Claims 7 to 9, characterised in that the said drum (10) has a cladding surface that is at least partly perforated (10a).

11. A device according to Claim 9 or Claim 10, characterised in that the said level of sub-atmospheric pressure is present only on the surface of the said drum.

12. A device according to any of Claims from 5 to 11, characterised in that the said transfer support (C) has at least one insert (23) selectively orientatable between at least a first angular position, reached at the said cutting means (11), and at least a second angular position, reached at the said substrate (CW), the angular extension of the orientation movement of the said at least one insert (23) between the said first and second positions determining the degree of inclination (α) of the said laminar elements (5) with respect to the said given direction.

13. A device according to Claim 12, characterised in that the said at least one insert (23) has orientation means (24 to 26) substantially configured as a cam.

14. A device according to Claim 7 and Claim 12, characterised in that the said at least one insert (23) corresponds to a respective portion of the cladding surface of the said drum (10).

15. A device according to Claim 14, characterised in that the said at least one insert (23) has an external surface of the generally cylindrical extension and a front flat surface (32) for receiving the said laminar elements.

16. A device according to Claim 7 and Claim 13, characterised in that a stationary structure (29) is provided within the said drum, which structure has external shaping (28) intended to co-operate with the said cam means (24 to 26).

17. A device according to Claim 16, characterised in that the said cam means include:
- a shaft (24) that controls the orientation of the respective insert (23),
- a crank arm (25) carried by the said shaft (24), and
- a cam follower element (26) carried by the said arm (25) and co-operating in a cam following relationship with the said shaping (28) on the said stationary structure (29).

18. A device according to Claim 17, characterised in that friction reduction means (27) are provided between the said cam element (26) and the said shaping (28).

19. A device according to any of Claims from 16 to 18, characterised in that the said drum (10) is moved by an associated shaft (22), and in that friction reduction means (30) are located between the said associated shaft (22) and the said stationary structure (29).

20. A device according to Claim 18 or Claim 19, characterised in that the said friction reduction means are bearings.

21. A device according to Claim 8 and Claim 12, characterised in that the said sub-atmospheric pressure generating means include at least one associated vacuum line (V2) leading to associated apertures (34) in at least one insert (23) in order to generate a pressure gradient across the surface of the said insert in order to hold the said laminar elements (5) against the said insert.

22. A device according to Claim 15 and Claim 21, characterised in that the said apertures (34) are located in the said front flat surface (32).

23. A device according to Claim 7 and Claim 21, characterised in that the said associated vacuum line (V2) includes a manifold (37) extending along the path of rotation of the cladding surface of the said drum (10), and in that the said at least one insert (23) includes an associated hole (36) able to put the said insert (23) selectively in communication with the said manifold (37) depending on the angular position reached by the said at least one insert (23) as the result of the movement of the said drum (10), so that the said at least one insert (23) is connected selectively to the said respective vacuum line (V) during the movement between the said cutting means (11) and the said transfer position (15).

24. A device according to Claim 23, characterised in that a pneumatic sealing element (38) is interposed between the said manifold (37) and the said hole (36).

25. A device according to any of Claims from 5 to 24, characterised in that the amplitude of the orientation movement imparted by the said orientation means (23) to the said laminar elements (5) is between approximately 10° and 20°.

26. A device according to any of Claims from 5 to 25, characterised in that the amplitude of the orientation movement imparted by the said orientation means (23) to the said laminar element is substantially equal to approximately 15°.

27. A sanitary article obtained using the method according to one of Claims from 1 to 4, and characterised in that it includes:
- a substrate (1) having a principal direction of extent (X1) corresponding to the said given direction, and
- at least one laminar element (5, 6) applied to the said substrate (6) and having a respective principal direction (X5) of stress resistance extending in an inclined direction (α) with respect to the said principal direction (X1) of the substrate.

28. An article according to Claim 27, characterised in that the said at least one laminar element (5) is rectangular in shape and is applied to the said substrate (1) with its larger sides inclined (α) with respect to the said principal direction (X1) of the substrate (1).

29. An article according to Claim 27 or Claim 28, characterised in that the article is a nappy, and the said at least one laminar element (5) is one of the closure tabs of the nappy (1).

30. An article according to Claim 29, characterised in that the article has applied thereto a plurality of the said laminar elements of which at least one (5) acts as a closure tab and at least another (6) acts as an attachment element for the said closure tab.

31. An article according to Claim 29 or Claim 30, characterised in that it includes at least a pair (5 and 6, respectively) of laminar elements applied to the said substrate (1) in a symmetrical position with respect to the said principal direction of extent (X1), and having similar functions.

32. An article according to Claim 30 and Claim 31, characterised in that it includes a pair of laminar elements (5) acting as closure tabs (5) and another pair of laminar elements (6) acting as attachment elements for the said closure elements (5), the laminar elements of both pairs being in an inclined position with respect to the said principal direction of extent (X1) of the substrate (1).
